# EUROPÄISCHE PATENTSCHRIFT

(11) **EP 2 634 257 B1**
(45) Veröffentlichungstag und Bekanntmachung des Hinweises auf die Patenterteilung: **26.09.2018**
(21) Anmeldenummer: 12001366.9
(22) Anmeldetag: 29.02.2012
(51) Int. Cl.: C12M 1/34, C12P 5/02, C12M 1/107, C12M 1/16

(54) **Verfahren zur Vergärung von Biomasse**
Fermentation method for biomass
Procédé de fermentation de biomasse

(43) Veröffentlichungstag der Anmeldung: 04.09.2013
(73) Patentinhaber: Kompoferm GmbH, 33428 Marienfeld (DE)
(72) Erfinder: Schürmann, Wilfried, DE-32584 Löhne (DE)
(74) Vertreter: Schober, Mirko

(56) Entgegenhaltungen:
- EP-A1- 0 106 857
- EP-A1- 2 149 005
- EP-A1- 2 428 558
- EP-A2- 1 447 613
- WO-A1-2014/079401
- WO-A2-2012/079735
- US-A- 4 437 987
- US-A- 4 439 315

## Beschreibung

Die Erfindung betrifft ein Verfahren zur Vergärung von fester stapelbarer Biomasse in einem Fermenter.

Bei Verfahren der in Rede stehenden Art wird Biomasse in einem diskontinuierlichen Prozess zu einem methanhaltigen Biogas vergoren. Bei der Biomasse handelt es sich dabei um feste, stapelbare Biomasse, wie beispielsweise Pflanzenabfälle verschiedenster Art. Dabei wird auch von fester stapelbarer Biomasse gesprochen, wenn diese während des Fermentationsprozesses zum großen oder sogar überwiegenden Teil eine eher pastöse Konsistenz annimmt. Von der Vergärung von fester Biomasse oder auch von Trockenfermentation wird auch im Sinne dieser Anmeldung gesprochen, sobald feste Biomasse direkt dem Fermenter zugeführt und nicht angemaischt wird, beispielsweise durch Zerkleinern und Versetzen mit Flüssigkeit, um eine zumindest zähflüssige und damit verfahrenstechnisch leichter handhabbare, insbesondere weil pumpbare Ausgangssubstanz zu erhalten. Stapelbar ist dabei so zu verstehen, dass es möglich ist, die Biomasse zu einem Haufwerk aufzuschichten.

Verfahren der in Rede stehenden Art beruhen darauf, dass zunächst die Biomasse in den Fermenter eingebracht wird. Der Eintrag der Biomasse in den Fermenter kann dabei durch fest installierte Beschickungsvorrichtungen oder durch mobile Geräte, wie beispielsweise Radlader erfolgen. Im weiteren Verfahren wird der Fermenter gasdicht verschlossen, um nach einer Anfahrphase während einer anaeroben Gärphase Methan haltiges Biogas durch die anaerobe Fermentation der Biomasse in dem Fermenter zu erzeugen. Das Biogas wird an ein Biogasverwertungssytem abgegeben, das an den Fermenter angeschlossen ist. Ist die anaerobe Gärphase beendet, was in der Regel dann der Fall ist, wenn nach wirtschaftlichen Gesichtspunkten die Biogasproduktion abgeklungen ist, so schließt sich an die Gärphase eine Abfahrphase an. Nach Beendigung der Abfahrphase kann der Fermenter geöffnet und der Gärrest entnommen werden, woraufhin sich der nächste Prozesszyklus, beginnend mit der erneuten Beladung des Fermenters mit neuer Biomasse anschließt.

Technisch bedingt, vor allem durch das Erfordernis, dass die Biomasse in den Fermenter ein- und ausgebracht werden muss, bildet sich oberhalb der Biomasse ein gasgefüllter Raum aus, der sogenannte Kopfraum des Fermenters. Wird der Prozess, insbesondere die Einleitung einzelner Prozessphasen, über die Steuerung der Zufuhr von Gasen, wie beispielsweise Luft oder Spülgasen in den Fermenter gesteuert, so stellt dieser Kopfraum ein als Mischvolumen wirkendes, störendes Totvolumen dar, da es während der Umsteuerprozesse zur Vermischung des noch aus einer Phase des Fermenterbetriebs stammenden, im Kopfraum eingeschlossenen Gases mit dem Prozessgas der nächsten Phase, welches in der Regel eine andere Zusammensetzung besitzt, kommt. Die so im Fermenter entstehenden Gasgemische weisen einen Methangehalt auf, der zu gering ist, um im Biogasverwertungssystem sinnvoll genutzt werden zu können und müssen daher über geeignete Schwachgassysteme entsorgt werden. Diese so generierten Methanverluste senken den Gesamtwirkungsgrad des Prozesses.

Aus der Druckschrift US 4,437,987 A ist ein Abwasserbehandlungstank bekannt, bei dem eine Membran dazu dient, einen konstanten Druck des Gases in einem Gasreservoir sicherzustellen. Die Funktionsweise ähnelt dabei der eines Gasometers.

Weiterhin offenbart die US 4,439,315 A einen kontinuierlichen Methangenerator, bei dem Schwankungen in der Gasproduktion durch eine Membran ausgeglichen werden. Diese kann sich zwischen verschiedenen Positionen bewegen und erlaubt so eine Volumenänderung des dortigen Fermenters.

Weiterhin offenbart die EP 2 428 558 A1 einen Fermenter, bei dem die Gasdichtigkeit durch eine flexible Hülle ermöglicht wird. Die Außenhülle dieses Fermenters besteht aus einer Kombination aus einer starren tragenden Struktur und einer flexiblen Hülle, was es ermöglicht, die dortigen Fermenter industriell vorzufertigen und nach Art von Containern zu modularen und/oder mobilen Biogasanlagen zusammen zu stellen.

Schließlich zeigt die WO 2012/079735 eine Biogasanlage, bei der die Möglichkeit besteht, auch erzeugte Biogase mit niedrigem Methangehalt einer energetischen Verwertung zuzuführen. Ermöglicht wird das durch die Zwischenspeicherung der erzeugten Biogase in Abhängigkeit von deren Methangehalt und die anschließende Vermischung dieser Biogase zu verwertbaren Gasmischungen.

Der Erfindung liegt daher die Aufgabe zugrunde, ein Verfahren zur Vergärung von Biomasse in einem Fermenter anzugeben, bei dem die Methanverluste beim Übergang von der Anfahr- in die Gär- und der Gär- in die Abfahrphase und der Stickstoff- und Sauerstoffeintrag ins Gassystem vermindert werden.

Die Aufgabe wird gelöst durch ein Verfahren mit den Merkmalen des Anspruchs 1. Die Merkmale der Unteransprüche betreffen vorteilhafte Verfahrensvarianten.

Erfindungsgemäß ist vorgesehen, vor dem Übergang der Anfahr- in die Gärphase und/oder dem Übergang der Gär- in die Abfahrphase das Volumen des Fermenters zu verkleinern. Dadurch wird das Gasvolumen, welches noch die Zusammensetzung der vorangegangenen Prozessphase hat und sich beim Umsteuern mit dem Gasgemisch der neuen Prozessphase mischen kann, verringert. Besonders effektiv lässt sich das erfindungsgemäße Verfahren gestalten, wenn die Volumenverkleinerung des Fermenters den Kopfraum des Fermenters betrifft. Hier lässt sich eine Verkleinerung des Fermentervolumens besonders gut umsetzen, da dieser Raum nicht von Biomasse eingenommen wird.

Die Verkleinerung des Fermentervolumens kann beispielsweise durch ein flexibles Trennelement erfolgen. Bei dem flexiblen Trennelement kann es sich um eine Membran handeln, worunter eine flexible, gasdichte Kunststoffschicht zu verstehen ist, die durch Änderung ihrer räumlichen Lage das Volumen des Fermenters, insbesondere des Kopfraums des Fermenters, verändert.

Zur Verkleinerung des Fermentervolumens kann dann beispielsweise die vom Innern des Fermenters abgewandte Seite des flexiblen Trennelements mit einem Druck, der größer ist als der Druck im Inneren des Fermenters, beaufschlagt werden. Das flexible Trennelement ändert daraufhin seine Lage in Richtung Biomasse und verdrängt so das im Fermenter vorhandene Gas und senkt das Fermentervolumen.

Um beim Ein- und/oder Ausbringen der Biomasse zu verhindern, dass das flexible Trennelement den Ein- und/oder Ausbringvorgang stört oder dabei beschädigt wird, ist es möglich, einen Druck, der niedriger ist als der Druck im Inneren des Fermenters, an der vom Inneren des Fermenters abgewandten Seite des flexiblen Trennelements anzulegen. Hierdurch wird das flexible Trennelement praktisch vom Inneren des Fermenters weg gesaugt. Wenn der Fermenter, wie häufig, eine feste, quaderförmige, garagenartige Betonhülle aufweist, so kann auf diese Weise das flexible Trennelement quasi an die Decke oder einen Wandbereich des Fermenters angesaugt werden, wo es dann flach anliegt und den Ein- und/oder Ausbringvorgang der Biomasse nicht behindert.

Vorteilhaft ist es dabei, während der Gärphase den Druck, mit dem die vom Inneren des Fermenters abgewandte Seite des flexiblen Trennelements beaufschlagt wird, so zu wählen, dass sich das flexible Trennelement frei bewegen kann. Dieser auch als Speicherbetrieb charakterisierte Betriebszustand hat den Vorteil, dass Schwankungen der Gasproduktion bzw. der Gasentnahme, welche normalerweise gesonderte Gasspeicher als Puffer erfordern, ausgeglichen werden können, da der Fermenter so praktisch ein flexibles Volumen aufweist, das in der Lage ist, Gasproduktions- bzw. Entnahmespitzen abzufangen, da sich das Volumen des Fermenters durch die frei bewegliche Membran abhängig von der im Fermenter anfallenden und der aus dieser abgeführten Gasmenge ändern kann, d.h. der Druck, mit dem die vom Fermenterinneren abgewandte Seite des flexiblen Trennelements beaufschlagt wird, ist insbesondere so gewählt, dass das flexible Trennelement weder seine maximale Ausdehnung ins Fermenterinnere aufweist, noch flach an der starren äußeren Hülle des Fermenters anliegt. Der Druck entspricht in etwa dem Druck im Fermenterinneren, er liegt lediglich minimal darunter, um das Eigengewicht des flexiblen Trennelements auszugleichen.

Vorteilhafterweise wird durch den Betrieb mit dem flexiblen, frei beweglichen Trennelement das Volumen des Fermenters während der Gärphase insbesondere im Bereich zwischen 20 und 30% des maximalen Fermentervolumens variiert. Durch diesen variablen Volumenanteil ergibt sich ein sinnvoll dimensionierter Pufferspeicher, der auf der einen Seite ausreicht, die im gewöhnlichen Betrieb auftretenden Spitzen abzufangen, auf der anderen Seite jedoch nicht mehr vom möglichen Nutzvolumen des Fermenters beansprucht als notwendig.

Vorteilhafterweise wird die Biomasse während der Gärphase mit einer Flüssigkeit beaufschlagt. Dabei handelt es sich vorteilhafterweise um Perkolat, d.h. eine mit Mikroorganismen angereicherte Flüssigkeit, mit der die Biomasse berieselt wird. Das Perkolat wird dabei vorzugsweise in einem Kreislauf durch den Fermenter gefahren und zwischen Entnahme aus dem Fermenter und Wiedereinbringen in den Fermenter weiter behandelt, insbesodere temperiert. Dabei wird das Perkolat im Fermenter vorzugsweise durch die Biomasse hindurch geleitet.

Um ein Kanalsystem zur besseren Perkolierbarkeit in der Biomasse zu erzeugen und aufrecht zu erhalten, ist es von Vorteil, den Fermenter so zu gestalten, dass die Biomasse von unten her mit einem Gas durchströmt werden kann. So ist es beispielsweise möglich, einen sogenannten Spigotboden vorzusehen, der eine Vielzahl kleiner Gasaustrittsöffnungen aufweist. Hierdurch ist es zum einen möglich, während der An- und/oder Abfahrphase Biomasse mit Frischluft zu durchströmen, andererseits ist es aber auch möglich, Spülvorgänge entweder mit einem methanhaltigen Biogas oder mit einem kohldioxydhaltigen Gas vorzunehmen.

Die Durchströmung des Fermenters und insbesondere auch der Biomasse während der Anfahrphase ist insbesondere deswegen von Vorteil, da bei der so entstehenden aeroben Umsetzung der Biomasse Wärme erzeugt wird. Abhängig davon, ob der Fermenter im mesophilen Temperaturbereich oder im thermophilen Temperaturbereich betrieben werden soll, wird die im Fermenter befindliche Biomasse so bis zum Erreichen einer Prozesstemperatur zwischen 36 und 52°C aufgeheizt, bevor von der Anfahr- in die Gärphase übergegangen wird. Dabei beträgt bei mesophilem Betrieb die Temperatur, bei der die Gärphase eingeleitet wird, 36 bis 42°C, vorzugsweise 39°C, bei thermophilem Betrieb 48 bis 52°C, vorzugsweise 50°C.

Eine Durchströmung des Fermenters und insbesondere der Biomasse mit Frischluft dient dazu, zum einen die anaerobe Vergärung zu stoppen, zum anderen die Gaszusammensetzung im Inneren des Fermenters, insbesondere den Methangehalt in einen Bereich zu bringen, bei dem ein Öffnen und Begehen bzw. Bewirtschaften des Fermenters gefahrlos möglich ist.

Im Hinblick auf eine wirtschaftliche Betriebsweise ist es sinnvoll, das Volumen des Kopfraums des Fermenters um mindestens 50%, vorzugsweise um mindestens 60% zu verkleinern, bzw. das Volumen des Fermenters um mindestens 22%, vorzugsweise um mindestens 26% des maximalen Fermentervolumens zu verkleinern. Bei diesen Volumenverhältnissen werden die Vorteile des erfindungsgemäßen Verfahrens spürbar, so dass sich die entsprechenden notwendigen Investitionen in die für die Durchführung des erfindungsgemäßen Verfahrens notwendige technische Ausrüstung auszahlen.

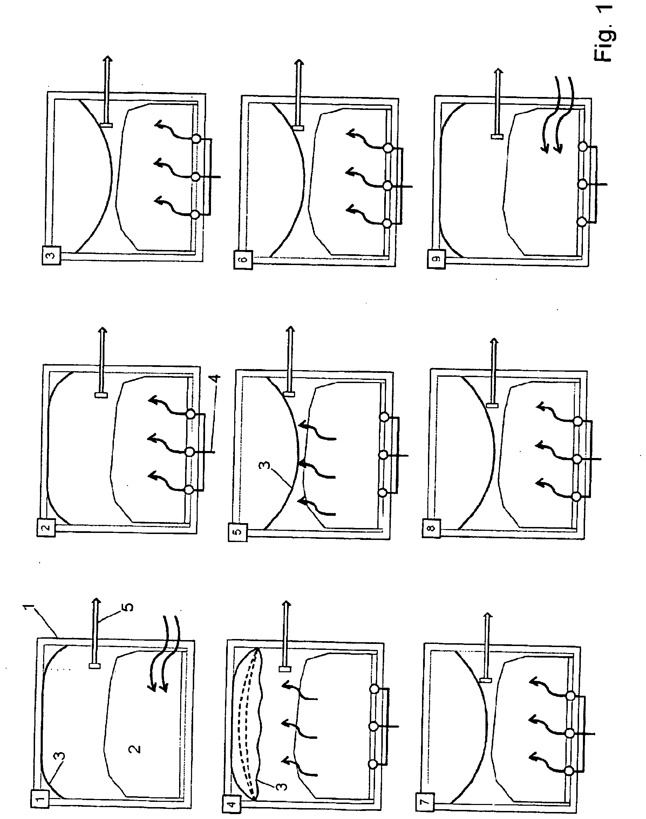

Die Erfindung wird im Folgenden anhand der Figur 1, die eine schematische Darstellung eines beispielhaften erfindungsgemäßen Verfahrens zeigt, näher erläutert.

Zu Beginn des Prozesses - Schritt 1 - ist das Fermentertor geöffnet. Das flexible Trennelement 3 liegt an der Decke des Fermenters 1 an, so dass das maximal mögliche Volumen des Fermenters 1 bei dessen Befüllung zur Verfügung steht. Die Biomasse 2 wird in den Fermenter 1 eingebracht. Der vom Fermenter 1 abgeführte Gasstrom 5 wird einer Abluftbehandlung zugeführt. Ein gewisser Luftwechsel mit Zuluft über das geöffnete Fermentertor ist beim Befüllen des Fermenters aus Arbeitsschutzgründen notwendig.

Schritt 2 verdeutlicht einen beispielhaften aeroben Anfahrbetrieb. Dabei wird Frischluft über den Gasverteilerboden 4 in den Fermenter 1 geleitet. Der vom Fermenter 1 abgeführte Gasstrom 5 weist aufgrund der aeroben Umsetzung der zugeführten Frischluft einen gewissen CO₂-Anteil auf, der jedoch noch unter dem Sauerstoffgehalt der Abluft liegt.

Schritt 3 zeigt den Fermenter 1 bei der Durchführung des erfindungsgemäßen Verfahrens am Ende der Anfahrphase. Durch das flexible Trennelement 3 wird das Fermentervolumen verkleinert und die Frischluftzufuhr wird gestoppt. Danach wird der vom Fermenter abgeführte Gasstrom 5 von dem Eingang der Abluftbehandlung auf den Eingang des Biogasverwertungssystems umgeschaltet. Dabei ist es möglich, zu diesem Zeitpunkt auch eine Spülung entweder mit einem Kohlendioxyd haltigen Gas, wobei hierunter auch Gase mit einem Kohldioxyd-Gehalt im Bereich weniger Vol.-% fallen, wie sie beispielsweise zu Beginn einer Gärphase entstehen oder eine Spülung mit einem methanhaltigen Biogas durchzuführen, um einen schnelleren Wechsel der Gaszusammensetzung und damit einen niedrigeren Verlust beim Übergang von der Anfahr- in die Gärphase zu ermöglichen. Besonders vorteilhaft ist dabei die Möglichkeit, nach dem Verkleinern des Fermentervolumens zunächst einen Spülvorgang mit einem Kohlendioxyd haltigen Gas, dann einen Spülvorgang mit einem methanhaltigen Biogas durchzuführen, bevor der aus dem Fermenter 1 abgeführte Gasstrom 5 auf den Eingang des Biogasverwertungssystem geschaltet wird.

Schritt 4 zeigt den Fermenter 1 während der Gärphase. Das flexible Trennelement 3 kann sich zwischen seinen Extremlagen frei bewegen, wodurch ein flexibles nutzbares Fermentervolumen entsteht und der Fermenter 1 gleichzeitig als Pufferspeicher für das Biogas dienen kann. Die Methankonzentration im Biogas steigt von dem Umsteuerzeitpunkt aus an, bis sie einen stationären Wert von etwa 50 bis 60 Vol.-% Methan erreicht. Auch während der Gärphase kann eine Spülung mit einem Methan haltigen Biogas, welches zu diesem Zweck auch im Kreislauf durch den Fermenter geführt werden kann, sinnvoll sein, um die Biomasse aufzulockern und so in der Biomasse Drainagekanäle für Perkolat zu schaffen und den Stofftransport bei der Vergärung zu beschleunigen.

Wie in Schritt 5 dargestellt, wird das Fermentervolumen kurz vor dem Übergang von der Gär- in die Abfahrphase verkleinert.

Schritt 6 zeigt das Einleiten der Abfahrphase durch das Einleiten von Frischluft oder einem Kohlendioxyd haltigen Gas in den Fermenter 1. Die Methankonzentration des aus dem Fermenter 1 abgeführten Gasstroms sinkt hierdurch kontinuierlich ab. Wird ein Methangehalt von 15 bis 25%, vorzugsweise 20% in dem aus dem Fermenter 1 abgeführten Gasstrom 5 erreicht, so wird vom Biogasverwertungssystem, welches beispielsweise ein Blockheizkraftwerk zur Verwertung des Biogases aufweisen kann, zunächst auf ein Schwachgassystem, welches beispielsweise eine Schwachgasfackel aufweisen kann, umgeschaltet (Schritt 7). Der Fermenter wird bei verkleinertem Volumen weiterhin entweder mit Frischluft oder mit einem Kohlendioxyd haltigen Gas gespült bis die Methankonzentration im vom Fermenter 1 abgeführten Gasstrom 5 auf 1 bis 4 Vol.-%, vorzugsweise auf 2,5 Vol.-% abgesunken ist. Dann wird im beispielhaften erfindungsgemäßen Verfahren der vom Fermenter 1 abgeführte Gasstrom 5 vom Schwachgassystem auf das Abluftsystem umgeschaltet. Der Methangehalt des Gasstroms 5 sinkt infolge weiterer Frischluftzufuhr in den Fermenter 1 weiter. Sobald ein Methangehalt < 1% erreicht ist, kann - wie in Schritt 9 gezeigt - das Fermentertor geöffnet werden, so dass die Zuluft über das geöffnete Fermentertor erfolgt und das Fermentervolumen auf das Maximalvolumen des Fermenters 1 vergrößert werden, um ein möglichst großes Fermentervolumen für das Entladen des Fermenters 1 bereit zu stellen.

Vorteilhaft ist es dabei, die während der Gärphasen aus einer Mehrzahl Fermenter abgeführten Biogasströme zur Homogenisierung der Gasqualität zusammenzuführen oder noch vorteilhafter durch den Gasraum eines Perkolatfermenters zu führen. Derartige Perkolatfermenter dienen dazu, aus dem aus den Fermentern abgeführten Perkolat weiteres Biogas zu gewinnen, das sich dann ebenfalls mit den aus den Fermentern abgeführten Biogasströmen mischt und so zu einem Gesamtbiogasstrom mit im Verhältnis zu den Gasströmen der einzelnen Fermenter gleich bleibender Zusammensetzung führt.

## Patentansprüche

1. Verfahren zur Vergärung von fester, stapelbarer Biomasse (2) in einem Fermenter (1), bei dem feste, stapelbare Biomasse (2), in einen Fermenter (1) eingebracht wird, nach einer Anfahrphase in einer anaeroben Gärphase zur Erzeugung von methanhaltigem Biogas vergoren wird, wobei sich an die Gärphase eine Abfahrphase anschließt, wobei ein Gasstrom (5) aus dem Fermenter (1) abgeführt wird, wobei die im Fermenter (1) befindliche Biomasse (2) auf eine Prozesstemperatur zwischen 36°C und 42°C oder zwischen 48°C und 52°C aufgeheizt wird, bevor von der Anfahr- in die Gärphase übergegangen wird,
**dadurch gekennzeichnet,**
**dass** beim Übergang der Anfahr- in die Gärphase vor dem Umschalten des aus dem Fermenter (1) abgeführten Gasstroms (5) vom Eingang eines Abluftsystems auf den Eingang eines Biogasverwertungssystems und/oder beim Übergang der Gär- in die Abfahrphase vor dem Umschalten des aus dem Fermenter (1) abgeführten Gasstroms (5) vom Eingang eines Biogasverwertungssystems auf den Eingang eines Schwachgassystems oder eines Abluftsystems das Volumen des Fermenters (1), verkleinert wird, wobei das Umschalten des aus dem Fermenter (1) abgeführten Gasstroms (5) vom Eingang eines Biogasverwertungssystems auf den Eingang eines Schwachgassystems bei Erreichen einer Methankonzentration im Kopfraum des Fermenters (1) von 15 bis 25 Vol.-% erfolgt.

2. Verfahren nach Anspruch 1,
**dadurch gekennzeichnet,**
**dass** die Verkleinerung des Fermentervolumens beim Übergang der Anfahr- in die Gärphase vor dem Umschalten des aus dem Fermenter (1) abgeführten Gasstroms (5) vom Eingang eines Abluftsystems auf den Eingang eines Blockheizkraftwerks erfolgt.

3. Verfahren nach Anspruch 1 oder 2,
**dadurch gekennzeichnet,**
**dass** eine Verkleinerung des Fermentervolumens beim Übergang der Gär- in die Abfahrphase vor dem Umschalten des aus dem Fermenter (1) abgeführten Gasstroms (5) vom Eingang eines Biogasverwertungssystems auf den Eingang eines Schwachgassystems oder eines Abluftsystems erfolgt.

4. Verfahren nach Anspruch 3,
**dadurch gekennzeichnet,**
**dass** das Umschalten des aus dem Fermenter (1) abgeführten Gasstroms (5) vom Eingang eines Biogasverwertungssystems auf den Eingang eines Schwachgassystems bei Erreichen einer Methankonzentration im Kopfraum des Fermenters (1) von 20 Vol. -% erfolgt.

5. Verfahren nach einem der vorigen Ansprüche,
**dadurch gekennzeichnet,**
**dass** die Verkleinerung des Fermentervolumens durch ein flexibles Trennelement (3) bewirkt wird.

6. Verfahren nach Anspruch 5,
**dadurch gekennzeichnet,**
**dass** das Verkleinern des Fermentervolumens durch Beaufschlagung der vom Inneren des Fermenters abgewandten Seite des flexiblen Trennelements mit einem Druck der größer ist als der Druck im Inneren des Fermenters erfolgt.

7. Verfahren nach Anspruch 5 oder 6,
**dadurch gekennzeichnet,**
**dass** beim Ein- und/oder Ausbringen der Biomasse (2) die vom Inneren des Fermenters (1) abgewandte Seite des flexiblen Trennelements (3) mit einem Druck beaufschlagt wird, der geringer ist als der Druck im Inneren des Fermenters (1).

8. Verfahren nach einem der Ansprüche 5 bis 7,
**dadurch gekennzeichnet,**
**dass** während der Gärphase die vom Inneren des Fermenters (1) abgewandte Seite des flexiblen Trennelements (3), insbesondere der Membran, mit einem Druck beaufschlagt wird, der relativ zum Innendruck des Fermenters so gewählt ist, dass sich das flexible Trennelement (3) frei bewegen kann.

9. Verfahren nach einem der vorigen Ansprüche,
**dadurch gekennzeichnet,**
**dass** das Volumen des Fermenters (1) während der Gärphase variiert wird.

10. Verfahren nach einem der vorigen Ansprüche,
**dadurch gekennzeichnet,**
**dass** die Biomasse (2) während der Gärphase mit Perkolat durchströmt wird.

11. Verfahren nach einem der vorigen Ansprüche,
**dadurch gekennzeichnet,**
**dass** der Fermenter (1) vor und/oder während der Gärphase mit Methan haltigem Biogas durchspült wird.

12. Verfahren nach einem der vorigen Ansprüche,
**dadurch gekennzeichnet,**
**dass** der Fermenter (1) beim Übergang der Anfahr- in die Gärphase und/oder beim Übergang der Gär- in die Abfahrphase mit Kohlendioxyd haltigem Gas durchspült wird.

13. Verfahren nach einem der vorigen Ansprüche,
**dadurch gekennzeichnet,**
**dass** der Fermenter (1) während der aeroben Anfahr- und/oder Abfahrphase mit Luft durchspült wird.

14. Verfahren nach einem der vorigen Ansprüche,
**dadurch gekennzeichnet,**
**dass** die aus einer Mehrzahl Fermenter (1) abgeführten Gasströme zur Homogenisierung der Gasqualität durch einen Gasraum eines Perkolatfermenters geführt werden.

15. Verfahren nach einem der vorigen Ansprüche,
**dadurch gekennzeichnet,**
**dass** das Volumen des Kopfraumes des Fermenters (1) um mindestens 50% des maximalen Kopfraumvolumens verkleinert wird und/oder das Volumen des Fermenters (1) um mindestens 22% des maximalen Fermentervolumens verkleinert wird.

## Claims

1. Method for fermenting solid stackable biomass (2) in a fermenter (1) in which solid stackable biomass (2) is introduced into a fermenter (1), is fermented after a start-up phase in an anaerobic fermenting phase to produce methane-containing biogas, wherein the fermenting phase is followed by a shut-down phase, wherein a gas stream (5) is discharged from the fermenter (1), wherein the biomass (2) located in the fermenter (1) is heated to a processing temperature between 36°C and 42°C or between 48°C and 52°C before being transferred from the start-up phase to the fermenting phase,
**characterised in that**
during the transition of the start-up phase into the fermenting phase the volume of the fermenter (1) is reduced prior to switching over the gas stream (5) discharged from the fermenter (1) from the entry of an exhaust air system to the entry of a biogas recovery system and/or during the transition of the fermenting phase into the shut-down phase prior to switching over the gas stream (5) discharged from the fermenter (1) from the entry of a biogas recovery system to the entry of a lean gas system or an exhaust gas system, wherein the switching over of the gas stream (5) discharged from the fermenter (1) from the entry of a biogas recovery system to the entry of a lean gas system takes place on reaching a methane concentration in the headroom of the fermenter (1) of 15 to 25% by volume.

2. Method according to claim 1
**characterised in that**
reducing the fermenter volume during the transition of the start-up phase into the fermenting phase takes place prior to switching over the gas stream (5) discharged from the fermenter (1) from the entry of an exhaust air system to the entry of a combined heat and power unit.

3. Method according to claim 1 or 2
**characterised in that**
reducing the fermenter volume during the transition of the fermenting phase into the shut-down phase take place prior to switching over the gas stream (5) discharged from the fermenter (1) from the entry of a biogas recovery system to the entry of a lean gas system or an exhaust air system.

4. Method according to claim 3
**characterised in that**
switching over the gas steam (5) discharged from the fermenter (1) from the entry of a biogas recovery system to the entry of a weak gas system takes place on reaching a methane concentration in the headroom of the fermenter (1) of 20 % by volume.

5. Method according to one of the preceding claims
**characterised in that**
the reduction in the fermenter volume is created by a flexible partition element (3).

6. Method according to claim 5
**characterised in that**
the reduction in the fermenter volume takes place by loading the side of the flexible partition element facing away from the inside of the fermenter with a pressure which is greater than the pressure inside the fermenter.

7. Method according to claim 5 or 6
**characterised in that**
when introducing and/or removing the biomass (2) the side of the flexible partition element (3) facing away from the inside of the fermenter (1) is loaded with a pressure which is less than the pressure inside the fermenter (1).

8. Method according to one of claims 5 to 7
**characterised in that**
during the fermenting phase the side of the flexible partition element (3), more particularly the membrane, facing away from the inside of the fermenter (1) is loaded with a pressure which is selected relative to the inner pressure of the fermenter so that the flexible partition element (3) can move freely.

9. Method according to one of the preceding claims
**characterised in that**
the volume of the fermenter (1) is varied during the fermenting phase.

10. Method according to one of the preceding claims
**characterised in that**
the biomass (2) is permeated with percolate during the fermenting phase.

11. Method according to one of the preceding claims
**characterised in that**
the fermenter (1) is flushed through with methane-containing biogas before and/or during the fermenting phase.

12. Method according to one of the preceding claims
**characterised in that**
the fermenter (1) is flushed through with carbon dioxide-containing gas during the transition of the start-up phase into the fermenting phase and/or during the transition of the fermenting phase into the shut-down phase.

13. Method according to one of the preceding claims
**characterised in that**
the fermenter (1) is flushed through with air during the aerobic start-up and/or shut-down phase.

14. Method according to one of the preceding claims
**characterised in that**
the gas streams discharged from a plurality of fermenters (1) are guided through a gas chamber of a percolate fermenter in order to homogenise the gas quality.

15. Method according to one of the preceding claims
**characterised in that**
the volume of the headroom of the fermenter (1) is reduced by at least 50% of the maximum headroom volume and/or the volume of the fermenter (1) is reduced by at least 22% of the maximum fermenter volume.

## Revendications

1. Procédé de fermentation de biomasse solide, empilable (2) dans un fermentateur (1), selon lequel de la biomasse solide, empilable (2) est amenée dans un fermentateur (1), après une phase de démarrage, est fermentée dans une phase de fermentation anaérobique pour la production d'un biogaz contenant du méthane, sachant qu'une phase de départ fait suite à la phase de fermentation, sachant qu'un flux de gaz (5) est évacué à partir du fermentateur (1), sachant que la biomasse (2), contenue dans le fermentateur (1) est chauffée à une température de processus située entre 36°C et 42°C ou entre 48°C et 52°C, avant de passer de la phase de démarrage à la phase de fermentation,
**caractérisé en ce que**,
lors du passage de la phase de démarrage à la phase de fermentation, avant que le flux de gaz (5) évacué hors du fermentateur (1) soit commuté de l'entrée d'un système d'échappement d'air à l'entrée d'un système d'exploitation du biogaz et / ou lors du passage de la phase de fermentation à la phase de départ avant que le flux de gaz (5), évacué hors du fermentateur (1), soit commuté de l'entrée d'un système d'exploitation du biogaz à l'entrée d'un système de gaz pauvre ou d'un système d'échappement d'air, le volume du fermentateur (1) est réduit, sachant que la commutation du flux de gaz (5), évacué hors du fermentateur (1), de l'entrée d'un système d'exploitation de biogaz à l'entrée d'un système de gaz pauvre st effectuée quand une concentration de méthane de 15 à 25 % en volume est atteinte dans l'espace de tête du fermentateur.

2. Procédé selon la revendication 1,
**caractérisé en ce que**
la réduction du volume du fermentateur est effectuée lors du passage de la phase de démarrage à la phase de fermentation, avant la commutation du flux de gaz (5), évacué hors du fermentateur (1), de l'entrée d'un système d'échappement d'air à l'entrée d'une centrale de cogénération.

3. Procédé selon revendication 1 ou 2,
**caractérisé en ce que**
la réduction du volume du fermentateur est effectué lors du passage de la phase de fermentation à la phase de départ, avant la commutation du flux de gaz (5), évacué hors du fermentateur (1), de l'entrée d'un système d'exploitation de biogaz à l'entrée d'un système d'échappement d'air.

4. Procédé selon la revendication 3,
**caractérisé en ce que**
la commutation du flux de gaz (5) en provenance du fermentateur (1) de l'entrée d'un système d'exploitation de biogaz à l'entrée d'un système de gaz pauvre, s'effectue quand une concentration de méthane de 20 % en volume est atteinte dans l'espace de tête du fermentateur (1).

5. Procédé selon l'une des revendications précédentes,
**caractérisé en ce que**,
la réduction du volume du fermentateur est produite par un élément de séparation (3) flexible.

6. Procédé selon la revendication 5,
**caractérisé en ce que**
la réduction du volume du fermentateur est effectuée par l'application, sur la face de l'élément de séparation flexible (3) opposée à l'intérieur du fermentateur (1}, une pression qui est supérieure à la pression à l'intérieur du fermentateur.

7. Procédé selon revendication 5 ou 6,
**caractérisé en ce que**,
lors de l'introduction et/ou l'extraction de la biomasse (2), la face de l'élément de séparation flexible (3), opposée à l'intérieur du fermentateur (1}, est soumise à une pression, qui est plus faible que la pression à l'intérieur du fermentateur (1).

8. Procédé selon l'une des revendications 5 à 7,
**caractérisé en ce que**,
pendant la phase de fermentation, la face de l'élément de séparation flexible (3) opposée à l'intérieur du fermentateur (1}, en particulier de la membrane, est soumise à une pression, qui, par rapport à la pression à l'intérieur du fermentateur, est choisie de manière à ce que l'élément flexible de séparation (3} puisse se mouvoir librement.

9. Procédé selon l'une des revendications précédentes,
**caractérisé en ce que**
le volume du fermentateur (1} est varié pendant la phase de fermentation.

10. Procédé selon l'une des revendications précédentes,
**caractérisé en ce que**
la biomasse (2) est traversée par un flux de percolât pendant la phase de fermentation.

11. Procédé selon l'une des revendications précédentes,
**caractérisé en ce que**
le fermentateur (1), avant et/ou pendant la phase de fermentation, est rincée avec du biogaz contenant du méthane.

12. Procédé selon l'une des revendications précédentes,
**caractérisé en ce que**
le fermentateur (1), lors du passage de la phase de démarrage à la phase de fermentation et/ou lors du passage de la phase de fermantation à la phase de départ, est rincé avec du gaz contenant du dioxyde de carbone.

13. Procédé selon l'une des revendications précédentes,
**caractérisé en ce que**
le fermentateur (1), pendant la phase de démarrage et/ou la phase de départ, est rincé avec de l'air.

14. Procédé selon l'une des revendications précédentes,
**caractérisé en ce que**
la biomasse (2) est traversée par un flux de percolât pendant la phase dé fermentation.

15. Procédé selon l'une des revendications précédentes,
**caractérisé en ce que**
le volume de l'espace de tête du fermentateur (1) est réduit d'au moins 50 % du volume maximal de l'espace de tête et/ou que le volume du fermentateur (1) est réduit d'au moins 22 % du volume maximale du fermentateur.
